# FASCICULE DE BREVET EUROPEEN

(11) **EP 1 738 801 B1**
(45) Date de publication et mention de la délivrance du brevet: **09.06.2010**
(21) Numéro de dépôt: 06116143.6
(22) Date de dépôt: 27.06.2006
(51) Int. Cl.: A61Q 5/10

(54) **Composition pour la décoloration et la coloration simultanées des fibres kératiniques comprenant un colorant anionique ou non ionique et un liquide inerte organique**
Mittel zum gleichzeitigen Bleichen oder Färben von Keratinfasern enthaltend einen anionischen oder nichtionischen Farbstoff und einen inerten Flüssigkeit
Agent for simultaneously bleaching and coloring of keratin fibres comprising an anionic or non-ionic dye and an inert organic liquid

(30) Priorité: 29.06.2005 FR 0551813
(43) Date de publication de la demande: 03.01.2007
(73) Titulaire: L'Oréal, 75008 Paris (FR)
(72) Inventeur: Kravtchenko, Sylvain, At Xintiandi 200021, Shanghaï (CN)
(74) Mandataire: Prevel, Estelle Nicole

(56) Documents cités:
- WO-A-01/28508
- WO-A-02/074270
- WO-A-2004/078150
- GB-A- 859 550
- US-A- 3 578 387
- US-A1- 2002 004 957

## Description

La présente invention a pour objet une composition pour la décoloration et la coloration simultanées des fibres kératiniques, et en particulier des fibres kératiniques humaines telles que les cheveux.

Lorsqu'une personne souhaite changer radicalement de couleur de cheveux, notamment lorsqu'elle souhaite obtenir une couleur plus claire que sa couleur d'origine, il est souvent nécessaire de procéder à une décoloration, et éventuellement à une coloration des cheveux. Pour ce faire, il existe plusieurs méthodes.

La première méthode consiste à utiliser des produits éclaircissants à base d'ammoniaque et de peroxyde d'hydrogène. Ces produits peuvent éventuellement contenir des colorants ce qui permet d'éclaircir et de colorer simultanément les cheveux. Toutefois, les performances éclaircissantes de ces produits restent limitées, plus particulièrement pour des applications sur des cheveux à fonds foncés naturels et / ou colorés.

La deuxième méthode consiste à appliquer sur les cheveux une composition éclaircissante à base de sels peroxygénés tels que les persulfates et d'agents alcalins dans laquelle a été ajouté du peroxyde d'hydrogène au moment de l'emploi, afin d'obtenir un éclaircissement plus important. Ce type de produit est très satisfaisant et plus adapté à des fonds foncés, mais il ne conduit qu'à une gamme très restreinte de reflets. Il est alors nécessaire de corriger la nuance obtenue en appliquant dans un deuxième temps un produit de coloration sur les cheveux. Ce procédé en deux étapes présente l'inconvénient d'être relativement long.

Pour pallier à cet inconvénient, il a été proposé, dans le brevet US 5 688 291, la demande de brevet WO 02/074270 et le modèle d'utilité DE 203 03 559, d'ajouter à ces produits éclaircissants des colorants, et en particulier des colorants directs de type anthraquinone, azo, triarylméthane, thiazine, quinone et nitro, qui sont pour certains stables dans ces milieux fortement oxydants. Cette méthode permet de colorer et de décolorer simultanément la fibre capillaire. Le niveau d'éclaircissement étant important, elle est particulièrement bien adaptée à des fonds foncés naturels et / ou colorés. Cependant, ces produits ont l'inconvénient de se présenter sous forme de poudres qui sont volatiles et de praticité réduite.

Le but de la présente invention est de fournir de nouvelles compositions pour la décoloration et la coloration simultanées des fibres kératiniques, et en particulier des fibres kératiniques humaines telles que les cheveux, particulièrement bien adaptées à des fonds foncés, qui sont faciles à utiliser et qui permettent d'obtenir des colorations chromatiques et tenaces.

Ce but est atteint avec la présente invention qui a pour objet une composition pour la décoloration et la coloration simultanées des fibres kératiniques comprenant :
- au moins un colorant direct choisi parmi les colorants anioniques et non ioniques, à l'exception du 7-(6'-méthylphénylazo)-1-acétamido-3,6-disulfo-8-hydroxy-naphtalène, des ortho nitro-anilines substituées en méta du groupement amino, de la quinoline, des dérivés quinoliniques, et de leurs sels d'addition ;
- au moins un liquide inerte organique ;
- au moins un sel peroxygéné ; et
- au moins un agent alcalin.

La composition conforme à la présente invention est particulièrement bien adaptée pour la décoloration et la coloration simultanées des cheveux foncés et est facile à utiliser. De plus, les colorants présentent une bonne stabilité dans la composition conforme à l'invention. La coloration obtenue est chromatique et, avec des concentrations adaptées en colorants selon l'invention, on peut obtenir une large palette de couleurs et de reflets.

Par ailleurs, cette coloration est résistante aux diverses agressions que peuvent subir les cheveux telles que les shampoings, les frottements, la lumière, les intempéries, la sueur et les déformations permanentes. Elle est aussi puissante, esthétique, et de plus peu sélective, c'est-à-dire qu'elle permet d'obtenir de faibles écarts entre différentes parties différemment sensibilisées d'un cheveu ou d'une chevelure.

La présente invention a également pour objet un procédé de décoloration et de coloration simultanées des fibres kératiniques mettant en oeuvre la composition conforme à l'invention, ainsi que des dispositifs à plusieurs compartiments pour la mise en oeuvre de ce procédé.

Un autre objet de la présente invention est l'utilisation de la composition conforme à l'invention pour la décoloration et la coloration simultanée des fibres kératiniques.

Les colorants anioniques utiles dans le cadre de l'invention peuvent être choisis parmi les colorants directs nitrés acides, les colorants azoïques acides, les colorants aziniques acides, les colorants triarylméthaniques acides, les colorants indoaminiques acides, les colorants naturels acides.

De préférence, les colorants anioniques utiles dans le cadre de l'invention sont choisis parmi les composés suivants :

| | |
|---|---|
| (C.I. 45380) | Acid Red 87 |
| (C.1. 10316) | Sel de sodium de l'acide 2,4-dinitro-1-naphtol-7-sulfonique |
| (C.I. 10383) | Acid Orange 3 |
| (C.I. 13015) | Acid Yellow 9 / Food Yellow 2 |
| (C.I. 14780) | Direct Red 45 / Food Red 13 |
| (C.I. 13711) | Acid Black 52 |
| (C.I. 13065) | Acid Yellow 36 |
| (C.I. 14700) | Sel de sodium de l'acide 1-hydroxy-2-(2',4'-xylyl-5-sufonatoazo)-naphtalène-4-sulfonique / Food Red 1 |
| (C.I. 14720) | Acid Red 14 / Food Red 3 / Mordant Blue 79 |
| (C. I. 14805) | Acid Brown 4 |
| (C.I. 15510) | Acid Orange 7 / Pigment Orange 17 / Solvent Orange 49 |
| (C.I. 15985) | Food Yellow 3 / Pigment Yellow 104 |
| (C.I. 16185) | Acid Red 27 / Food Red 9 |
| (C.I. 16230) | Acid Orange 10 / Food Orange 4 |
| (C.I. 16250) | Acid Red 44 |
| (C.I. 17200) | Acid Red 33 / Food Red 12 |
| (C.I. 15685) | Acid Red 184 |
| (C.I. 19125) | Acid Violet 3 |
| (C.1.18055) | Sel de sodium de l'acide 1-hydroxy-2-(4'-acétamido phénylazo)-8-acétamido-naphtalène-3,6-disulfonique / Acid Violet 7 / Food Red 11 |
| (C.I. 18130) | Acid Red 135 |
| (C.I. 19130) | Acid Yellow 27 |
| (C.I. 19140) | Acid Yellow 23 / Food Yellow 4 |
| (C.I. 20170) | 4'-(sulfonato-2",4"-diméthyl)-bis-(2,6-phénylazo)-1,3-dihydroxy benzène / Acid Orange 24 |
| (C.I. 20470) | Sel de sodium de l'acide 1-amino-2-(4'-nitrophénylazo)-7-phénylazo-8-hydroxy-naphtalène-3,6-disulfonique / Acid Black 1 |
| (C.I. 23266) | (4-((4-méthylphényl) sulfonyloxy)-phénylazo)2,2'-diméthyl-4-((2-hydroxy-5,8-disulfonato)naphtylazo)biphényle / Acid Red 111 |
| (C.I. 27755) | Food Black 2 |
| (C.I. 25440) | 1-(4'-sulfonatophénylazo)-4-((2"-hydroxy-3"-acétylamino-6",8"-disulfonato)naphtylazo)-6-sulfonatonaphtalène (sel tétrasodique) / Food Black 1 |
| (C.I. 42080) | Acide 4-β-hydroxyéthylamino-3-nitrobenzènesulfonique |
| (C.I. 42090) | Acid Blue 9 |
| (C.I. 60730) | Acid Violet 43 |
| (C.I. 61570) | Acid Green 25 |
| (C.I. 62045) | Sel de sodium de l'acide 1-amino-4-cyclohexylamino-9,10-anthraquinone 2-sulfonique / Acid Blue 62 |
| (C.I. 62105) | Acid Blue 78 |
| (C.I. 14710) | Sel de sodium de l'acide 4-hydroxy-3((2-méthoxy phényl)-azo)-1-naphtalène sulfonique / Acid Red 4 |
| | Acide 2-pipéridino 5-nitro benzène sulfonique |
| | Acide 2(4'-N,N(2"-hydroxyéthyl)amino-2'-nitro)aniline éthane sulfonique |
| | Acide 4-β-hydroxyéthylamino-3-nitrobenzène sulfonique |
| (C.I. 42640) | Acid Violet 49 |
| (C.I. 42080) | Acid Blue 7 |
| | Acid Blue 156 |
| | Acid Blue 317 |
| (C.I. 58005) | Sel de sodium du 1,2-dihydroxy-3-sulfo-anthraquinone / Mordant Red 3 |
| (C.I. 62055) | Sel de sodium de l'acide 1-amino-9,10-dihydro-9,10-dioxo-4-(phénylamino) 2-anthracène sulfonique / Acid Blue 25 |
| (C.I. 14710) | Sel de sodium de l'acide 4-hydroxy-3-((2-méthoxyphényl)-azo)-1-naphtalène sulfonique / Acid Red 4 |

La plupart de ces colorants sont décrits en particulier dans le Color Index publié par The Society of Dyers and Colorists, P.O. Box 244, Perkin House, 82 Grattan Road, Bradford, Yorkshire, BD1 2JBN England.

Les colorants anioniques plus particulièrement préférés sont les colorants désignés dans le Color Index sous le code C.I. 58005 (sel monosodique de l'acide 1,2-dihydroxy-9,10-anthraquinone-3-sulfonique), C.I. 60730 (sel monosodique de l'acide 2-[(9,10-dihydro-4-hydroxy-9,10-dioxo-1-anthracényl)-amino]-5-méthyl-benzène sulfonique), C.I. 15510 (sel monosodique de l'acide 4-[(2-hydroxy-1-naphtalényl)-azo]-benzène sulfonique), C.I. 15985 (sel disodique de l'acide 6-hydroxy-5-[(4-sulfophényl)-azo]-2-naphtalène sulfonique), C.I. 17200 (sel disodique de l'acide 5-amino-4-hydroxy-3-(phénylazo)-2,7-naphtalène disulfonique), C.I. 20470 (sel disodique de l'acide 1-amino-2-(4'-nitrophénylazo)-7-phénylazo-8-hydroxy-3,6-naphtalène disulfonique), C.I. 42090 (sel disodique du N-éthyl-N-[4-[[4-[éthyl[3-sulfophényl)-méthyl]-amino]-phényl](2-sulfophényl)-méthylène]-2,5-cyclohexadien-1-ylidène]-3-sulfobenzenemethanaminium hydroxyde, sel interne), C.I. 61570 ( sel disodique de l'acide 2,2'-[(9,10-dihydro-9,10-dioxo-1,4-anthracènediyl)-diimino]-bis-[5-méthyl]-benzène sulfonique.

Le ou les colorants anioniques pouvant être utilisés dans le cadre de la présente invention sont également préférentiellement choisis parmi les composés suivants :
- l'acide 4-(β-hydroxyéthyl)amino-3-nitro-benzènesulfonique,
- l'acide 4-N-éthylamino-3-nitrobenzoique ;
- l'acide 2-pipéridino-5-nitrobenzoique ;
- l'acide 4-amino-2-nitrodiphénylamine-2' carboxylique ;
- l'acide 4-amino-4'-diméthylamino-2-nitrodiphénylamine-2'-carboxylique ;
- l'acide 3-oxo-6-hydroxy-9-carboxy-phényl xanthylium.

Le ou les colorants non ioniques utiles dans le cadre de l'invention peuvent être choisis parmi les colorants non ioniques benzéniques nitrés, les colorants non ioniques azoïques, azométhiniques, méthiniques, anthraquinoniques, naphtoquinoniques, benzoquinoniques, phénotiaziniques indigoïdes, xanthéniques, phénanthridiniques, phtalocyanines, ceux dérivés du triarylméthane, seuls ou en mélanges.

Ils peuvent par exemple être choisis parmi les colorants benzéniques nitrés rouges ou orangés, par exemples les composés suivants :
- le 1-hydroxy-3-nitro-4-N-(y-hydroxypropyl)amino benzène,
- le N-(β-hydroxyéthyl)amino-3-nitro-4-amino benzène,
- le 1-hydroxy-3-nitro-4-N-(β-hydroxyéthyl)amino benzène,
- le 1,4-diamino-2-nitrobenzène,
- le 1-amino-2-nitro-4-méthylamino benzène,
- la N-(β-hydroxyéthyl)-2-nitro-paraphénylènediamine,
- la 2-nitro-4-amino-diphénylamine,
- le1-amino-3-nitro-6-hydroxybenzène.
- le 1-(β-aminoéthyl)amino-2-nitro-4-(β-hydroxyéthyloxy) benzène,
- le 1-(β, γ-dihydroxypropyl)oxy-3-nitro-4-(β-hydroxyéthyl)amino benzène,
- le 1-hydroxy-3-nitro-4-aminobenzène,
- le 1-hydroxy-2-amino-4,6-dinitrobenzène,
- le 1-méthoxy-3-nitro-4-(β-hydroxyéthyl)amino benzène,
- la 2-nitro-4'-hydroxydiphénylamine.

Ils peuvent également être choisis parmi les colorants directs benzéniques nitrés jaunes et jaune-verts, tels que les composés suivants :
- le 1-(β-hydroxyéthyl)amino-2-méthoxy-4-nitrobenzène,
- le 1-amino-2-nitro-6-méthyl-benzène,
- le 1-(β-hydroxyéthyl)amino-2-hydroxy-4-nitrobenzène,
- la N-(β-hydroxyéthyl)-2-nitro-4-trifluorométhylaniline,
- le 4-(β-hydroxyéthyl)amino-3-nitro-chlorobenzène,
- le 4-(β-hydroxyéthyl)amino-3-nitro-méthylbenzène,
- le 4-(β,γ -dihydroxypropyl)amino-3-nitro-trifluorométhylbenzène,
- le 1-(β-uréidoéthyl)amino-4-nitrobenzène,
- le 1-hydroxy-2-amino-5-nitrobenzène,
- le 1-amino-2-[tris(hydroxyméthyl)méthyl]amino-5-nitro-benzène,
- le 1-(β-hydroxyéthyl)amino-2-nitrobenzène,
- le 4-(β-hydroxyéthyl)amino-3-nitrobenzamide.

Ils peuvent aussi être choisis parmi les colorants directs benzéniques nitrés bleus ou violets. On peut par exemple citer les composés suivants :
- le 1-(β-hydroxyéthyl)amino-4-N,N-bis-(β-hydroxyéthyl)amino 2-nitrobenzène,
- le 1-(γ-hydroxypropyl)amino 4-N,N-bis-(β-hydroxyéthyl)amino 2-nitrobenzène,
- le 1-(β-hydroxyéthyl)amino 4-(N-méthyl, N-β-hydroxyéthyl)amino 2-nitrobenzène,
- le 1-(β-hydroxyéthyl)amino 4-(N-éthyl, N-β-hydroxyéthyl)amino 2-nitrobenzène,
- le 1-(β,γ-dihydroxypropyl)amino 4-(N-éthyl, N-β-hydroxyéthyl)amino 2-nitrobenzène,
- les 2-nitroparaphénylènediamines de formule suivante : 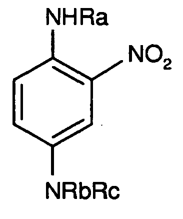 dans laquelle :
   - Rb représente un radical alkyle en C₁-C₄, un radical β-hydroxyéthyle ou β-hydroxypropyle ou γ-hydroxypropyle ;
   - Ra et Rc, identiques ou différents, représentent un radical β-hydroxyéthyle, -β-hydroxypropyle, γ-hydroxypropyle, ou β,γ-dihydroxypropyle, l'un au moins des radicaux Rb, Rc ou Ra représentant un radical γ-hydroxypropyle et Rb et Rc ne pouvant désigner simultanément un radical β-hydroxyéthyle lorsque Ra est un radical γ-hydroxypropyle, telles que ceux décrits dans le brevet français FR 2 692 572.

A titre de colorants non ioniques, on peut aussi citer les colorants suivants : le Disperse Orange 3; le Disperse Red 17; le Disperse Violet 4 ; le Disperse Violet 8 ; le Disperse Blue 1 ; le Disperse Red 15 ; le Solvent Violet 13 ; le Solvent Violet 11 ; le Disperse Blue 3 ; le Disperse Blue 7 ; le Disperse Red 11 ; le Natural Brown 7 ; le Disperse Black 9 ; le Disperse Violet 15 ; le Natural Orange 6 ; la 2-hydroxy 3-méthoxy 1,4-naphtoquinone ; la 3-hydroxy 2-méthyl 1,4-naphtoquinone ou le phtiocol ; la 3,6-dihydroxy 5-méthoxy p-toluquinone ou la spinulosine ; le HC Blue 14.

De préférence, le ou les colorants non ioniques sont choisis parmi le 1-hydroxy-3-nitro-4-N-(y-hydroxypropyl)amino benzène ; le N-(β-hydroxyéthyl)amino-3-nitro-4-amino benzène ; le 1-hydroxy-3-nitro-4-N-(β-hydroxyéthyl)amino benzène ; le 1,4-diamino-2-nitrobenzène ; le 1-amino-2-nitro-4-méthylamino benzène; la N-(β-hydroxyéthyl)-2-nitro-paraphénylènediamine ; la 2-nitro-4-amino-diphénylamine ; le 1-amino-3-nitro-6-hydroxybenzène ; le 1-(β-aminoéthyl)amino-2-nitro-4-(β-hydroxyéthyloxy) benzène; le 1-(β, γ-dihydroxypropyl)oxy-3-nitro-4-(β-hydroxyéthyl)amino benzène ; le 1-hydroxy-3-nitro-4-aminobenzène ; le 1-hydroxy-2-amino-4,6-dinitrobenzène ; le 1-méthoxy-3-nitro-4-(β-hydroxyéthyl)amino benzène ; la 2-nitro-4'-hydroxydiphénylamine ; le 1-(β-hydroxyéthyl)amino-2-méthoxy-4-nitrobenzène ; le 1-amino-2-nitro-6-méthyl-benzène ; le 1-(β-hydroxyéthyl)amino-2-hydroxy-4-nitrobenzène ; la N-(β-hydroxyéthyl)-2-nitro-4-trifluorométhylaniline ; le 4-(β-hydroxyéthyl)amino-3-nitro-chlorobenzène ; le 4-(β-hydroxyéthyl)amino-3-nitro-méthylbenzène ; le 4-(β,γ -dihydroxypropyl)amino-3-nitro-trifluorométhylbenzène; le 1-(β-uréidoéthyl)amino-4-nitrobenzène ; le 1-hydroxy-2-amino-5-nitrobenzène ; le 1-amino-2-[tris(hydroxyméthyl)méthyl]amino-5-nitro-benzène ; le 1-(β-hydroxyéthyl)amino-2-nitrobenzène ; le 4-(β-hydroxyéthyl)amino-3-nitrobenzamide; le 1-(β-hydroxyéthyl)amino-4-N,N-bis-(β-hydroxyéthyl)amino 2-nitrobenzène ; le 1-(γ-hydroxypropyl)amino 4-N,N-bis-(β-hydroxyéthyl)amino 2-nitrobenzène ; le 1-(β-hydroxyéthyl)amino 4-(N-méthyl, N-β-hydroxyéthyl)amino 2-nitrobenzène ; le 1-(β-hydroxyéthyl)amino 4-(N-éthyl, N-β-hydroxyéthyl)amino 2-nitrobenzène ; le 1-(β,γ-dihydroxypropyl)amino 4-(N-éthyl, N-β-hydroxyéthyl)amino 2-nitrobenzène ; le Disperse Orange 3 ; le Disperse Red 17 ; le Disperse Violet 4 ; le Disperse Violet 8 ; le Disperse Blue 1 ; le Disperse Red 15 ; le Solvent Violet 13 ; le Solvent Violet 11 ; le Disperse Blue 3 ; le Disperse Blue 7 ; le Disperse Red 11 ; le Natural Brown 7 ; le Disperse Black 9 ; le Disperse Violet 15 ; le Natural Orange 6 ; la 2-hydroxy 3-méthoxy 1,4-naphtoquinone ; la 3-hydroxy 2-méthyl 1,4-naphtoquinone ou le phtiocol ; la 3,6-dihydroxy 5-méthoxy p-toluquinone ou la spinulosine ; le HC Blue 14.

La concentration en colorants anioniques et / ou non ioniques dans la composition conforme à l'invention est généralement comprise entre 0,0001 et 10 % en poids, de préférence entre 0,001 et 8 %, et encore plus préféntiellement entre 0,01 et 5 % en poids du poids total de la composition.

Par phase liquide, on entend au sens de la présente invention toute phase capable d'écoulement à température ambiante, généralement entre 15 °C et 40 °C, et à pression atmosphérique, sous l'action de son propre poids.

Par liquide inerte organique, on entend au sens de la présente invention un liquide organique chimiquement inerte vis-à-vis du peroxyde d'hydrogène. Dans le cadre de l'invention, un liquide est inerte si la dégradation du peroxyde d'hydrogène en présence de ce liquide est inférieure à 25 % après 15 heures à 100 °C.

A titre d'exemple de liquide inerte organique, on peut citer les polydécènes de formule C₁₀ₙH_{[(20n)+2]} dans laquelle n varie de 3 à 9 et de préférence de 3 à 7, les esters d'alcools gras ou d'acides gras, les esters ou di-esters de sucres d'acides gras en C₁₂-C₂₄, les éthers cycliques ou les esters cycliques, les huiles de silicone, les huiles minérales ou les huiles végétales, ou leurs mélanges.

Les composés de formule C₁₀ₙ H_{[(20n)+2]} avec n variant de 3 à 9 répondent à l'appellation "polydécène" du Dictionnaire CTFA 7ème édition 1997 de la Cosmetic, Toiletry and Fragrance Association, USA, ainsi qu'à la même appellation I.N.C.I. aux USA et en Europe. Ce sont des produits d'hydrogénation des poly-1-décènes.

Parmi ces composés, on préfère selon l'invention ceux pour lesquels dans la formule, n varie de 3 à 7.

On peut citer à titre d'exemple le produit vendu sous la dénomination Silkflo^{®} 366 NF Polydecene par la société Amoco Chemical, ceux vendus sous la dénomination Nexbase® 2002 FG, 2004 FG, 2006 FG et 2008 FG par la société Fortum.

En ce qui concerne les esters d'alcools gras ou d'acides gras, on peut citer à titre d'exemple :
- les esters de monoalcools inférieurs saturés linéaires ou ramifiés en C₃-C₆, avec des acides gras monofonctionnels en C₁₂-C₂₄, ces derniers pouvant être linéaires ou ramifiés, saturés ou insaturés et choisis notamment parmi les oléates, laurates, palmitates, myristates, béhénates, cocoates, stéarates, linoléates, linolénates, caprates, arachidonates, ou leurs mélanges comme notamment les oléo-palmitates, oléo-stéarates, palmito-stéarates. Parmi ces esters, on préfère plus particulièrement utiliser le palmitate d'isopropyle, le myristate d'isopropyle et le stéarate d'octyl dodécyle,
- les esters de monoalcools linéaires ou ramifiés en C₃-C₈, avec des acides gras bifonctionnels en C₈-C₂₄, ces derniers pouvant être linéaires ou ramifiés, saturés ou insaturés, comme par exemple le di-ester isopropylique de l'acide sébacique, appelé aussi sébaçate de diisopropyle,
- les esters de monoalcools linéaires ou ramifiés en C₃-C₈, avec des acides gras bifonctionnels en C₂-C₈, ces derniers pouvant être linéaires ou ramifiés, saturés ou insaturés, comme par exemple l'adipate de di-octyle et le maléate de di-caprylyle,
- l'ester d'un acide trifonctionnnel comme le citrate de tri-éthyle.

En ce qui concerne les esters et di-esters de sucres d'acides gras en C₁₂-C₂₄, on entend par "sucre" des composés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres utilisables selon l'invention, on peut citer par exemple le sucrose (ou saccharose), le glucose, le galactose, le ribose, le fuctose, le maltose, le fructose, le mannose, l'arabinose, le xylose, le lactose, et leurs dérivés notamment alkylés, tels que les dérivés méthylés comme le méthylglucose.

Les esters de sucres et d'acides gras utilisables selon l'invention peuvent être choisis notamment dans le groupe comprenant les esters ou mélanges d'esters de sucres décrits ci-avant et d'acides gras en C₁₂-C₂₄, linéaires ou ramifiés, saturés ou insaturés.

Les esters peuvent être choisis parmi les mono-, di-, tri- et tétra-esters, les polyesters et leurs mélanges.

Ces esters peuvent être par exemple choisis parmi les oléates, laurates, palmitates, myristates, béhénates, cocoates, stéarates, linoléates, linolénates, caprates, arachidonates, ou leurs mélanges comme notamment les esters mixtes oléo-palmitates, oléo-stéarates, palmito-stéarates.

Plus particulièrement, on préfère utiliser les mono- et di- esters et notamment les mono- ou di- oléates, stéarates, béhénates, oléopalmitates, linoléates, linolénates, oléostéarates, de saccharose, de glucose ou de méthylglucose.

On peut citer à titre d'exemple le produit vendu sous la dénomination Glucate® DO par la société Amerchol, qui est un dioléate de méthylglucose.

On peut aussi citer à titre d'exemples d'esters ou de mélanges d'esters de sucre d'acide gras :
- les produits vendus sous les dénominations F160, F140, F110, F90, F70, SL40 par la société Crodesta, désignant respectivement les palmito-stéarates de sucrose formés de 73 % de monoester et 27 % de di- et triester, de 61 % de monoester et 39 % de di-, tri-, et tétra-ester, de 52 % de monoester et 48 % de di-, tri-, et tétra-ester, de 45 % de monoester et 55 % de di-, tri-, et tétra-ester, de 39 % de monoester et 61 % de di-, tri-, et tétra-ester, et le mono-laurate de sucrose ;
- les produits vendus sous la dénomination Ryoto Sugar Esters par exemple référencés B370 et correspondant au béhénate de saccharose formé de 20 % de monoester et 80 % de di-triester-polyester ;
- le mono-di-palmito-stéarate de sucrose commercialisé par la société Goldschmidt sous la dénomination Tegosoft® PSE.

En ce qui concerne les ethers cycliques et esters cycliques, conviennent notamment la γ-butyrolactone, le diméthyl isosorbide, ou le diisopropyl isosorbide.

Les huiles de silicone peuvent aussi être employées comme liquide organique inerte.

Plus particulièrement, les huiles de silicone convenables sont des fluides de silicones liquides et non volatiles de viscosité inférieure ou égale à 10 000 mPa.s à 25 °C, la viscosité des silicones étant mesurée selon la norme ASTM 445 Appendice C.

Les huiles de silicone sont définies plus en détail dans l'ouvrage de Walter NOLL "Chemistry and Technology of Silicones" (1968) - Academic Press.

Parmi les huiles de silicone utilisables selon l'invention, on peut citer notamment les huiles de silicones vendues sous les dénominations DC-200 fluid - 5 mPa.s, DC-200 fluid - 20 mPa.s, DC-200 fluid - 350 mPa.s, DC-200 fluid - 1000 mPa.s, DC-200 fluid - 10 000 mPa.s par la société Dow Corning.

Les huiles minérales peuvent aussi être utilisées comme liquide inerte organique, comme par exemple l'huile de paraffine.

Les huiles végétales peuvent aussi convenir, et notamment l'huile d'avocat, l'huile d'olive ou la cire liquide de jojoba.

De préférence, le ou les liquides inertes organiques sont choisis dans le groupe formé par les polydécènes de formule C₁₀ₙH_{[(20n)+2]} dans laquelle n varie de 3 à 9 et de préférence de 3 à 7, les esters d'alcools gras ou d'acides gras, et leurs mélanges.

La teneur en liquides inertes organiques varie généralement de 5 à 60 % en poids, de préférence de 10 à 50 % en poids par rapport au poids de la pâte anhydre, et encore plus préférentiellement de 15 à 45 %.

La composition conforme à l'invention se présente sous forme de pâte.

Le ou les sels peroxygénés utiles à l'invention sont par exemple choisis parmi les persulfates, les perborates, les percarbonates, les peroxydes de métaux alcalins ou alcalino-terreux, et leurs mélanges. De préférence, on utilisera les persulfates et leurs mélanges, par exemple les persulfates de sodium, de potassium et d'ammonium, et leurs mélanges.

La concentration en sels peroxygénés dans la composition conforme à l'invention est généralement comprise entre 1 et 70 % en poids, et de préférence entre 20 et 60 % en poids du poids total de la composition.

Le ou les agents alcalins utiles dans la composition de la présente invention sont par exemple choisis parmi l'urée, les sels d'ammonium comme le chlorure d'ammonium, le sulfate d'ammonium, le phosphate d'ammonium ou le nitrate d'ammonium, les silicates, les phosphates ou les carbonates de métaux alcalins ou alcalino-terreux, tels que le lithium, le sodium, le potassium, le magnésium, le calcium, le baryum, et leurs mélanges. De préférence, le ou les agents alcalins sont choisis parmi le chlorure d'ammonium, les silicates, les carbonates, et leurs mélanges.

La concentration en agents alcalins dans la composition conforme à l'invention est généralement comprise entre 0,01 et 40 % en poids, et de préférence entre 0,1 et 30 % en poids du poids total de la composition.

Selon un mode de réalisation particulier de l'invention, la composition conforme à l'invention est anhydre.

Dans le cadre de la présente invention, une composition est anhydre lorsqu'elle présente une teneur en eau inférieure à 1 % en poids, et de préférence inférieure à 0,5 % en poids par rapport au poids total de la composition.

Selon un autre mode de réalisation particulier de l'invention, la composition conforme à l'invention est aqueuse. Elle peut alors de plus comprendre du peroxyde d'hydrogène.

Dans ce cas-là, la composition conforme à l'invention est prête à l'emploi et résulte du mélange d'une composition anhydre conforme à l'invention avec une composition aqueuse comprenant ou non du peroxyde d'hydrogène. Son pH est généralement compris entre les valeurs 3 et 11. Il est de préférence compris entre 7 et 11.

La composition conforme à la présente invention peut également comprendre divers additifs classiquement utilisés en cosmétique.

La composition conforme à la présente invention peut ainsi comprendre des agents épaississants minéraux ou organiques, et en particulier des polymères épaississants associatis ou non, anioniques, cationiques, non ioniques ou amphotères, des charges telles que des argiles, des liants tels que la vinylpyrrolidone, des lubrifiants comme les stéarates de polyol ou les stéarates de métaux alcalins ou alcalino-terreux, des silices hydrophiles ou hydrophobes, des pigments, des colorants autres que ceux de la présente invention, des agents matifiants comme les oxydes de titane ou encore des agents tensioactifs anioniques, non ioniques, cationiques, amphotères ou zwittérioniques, des agents antioxydants, des agents de pénétration, des agents séquestrants, des tampons, des agents dispersants, des agents filmogènes, des agents conservateurs, des agents opacifiants, des vitamines, des parfums, des polymères anioniques, cationiques, non ioniques, amphotères ou zwittérioniques, des céramides, des agents de conditionnement tels que par exemple des silicones volatiles ou non volatiles, modifiées ou non modifiées.

Dans le cas où la composition conforme à l'invention comprend du peroxyde d'hydrogène, elle peut également comprendre des agents de contrôle du dégagement d'oxygène tels que le carbonate ou l'oxyde de magnésium.

Les additifs et les agents de contrôle du dégagement d'oxygène tels que définis précédemment peuvent être présents en quantité comprise pour chacun d'eux entre 0,01 et 40 % en poids, de préférence entre 0,1 et 30 % en poids par rapport au poids total de la composition.

Bien entendu, l'homme de l'art veillera à choisir ce ou ces éventuels composés complémentaires de manière telle que les propriétés avantageuses attachées intrinsèquement à la composition conforme à l'invention ne soient pas, ou substantiellement pas, altérées par la ou les adjonctions envisagées.

Le procédé de décoloration et de coloration simultanées conforme à la présente invention consiste à appliquer sur les fibres kératiniques une composition anhydre conforme à l'invention telle que définie précédemment en présence d'une composition aqueuse comprenant ou non du peroxyde d'hydrogène. La composition aqueuse comprenant ou non du peroxyde d'hydrogène peut être ajoutée à la composition anhydre juste au moment de l'emploi. Elle peut aussi être appliquée simultanément ou séquentiellement à la composition anhydre.

La présente invention a également pour objet un dispositif à plusieurs compartiments, caractérisé par le fait qu'il contient au moins deux compositions dont le mélange conduit à une composition aqueuse conforme à l'invention telle que définie précédemment.

Selon un mode de réalisation particulier de l'invention, le dispositif conforme à invention comporte un premier compartiment qui contient une composition (A) comprenant, dans un milieu approprié pour la teinture, au moins un colorant anionique ou non ionique et au moins un liquide inerte organique tels que définis précédemment, un deuxième compartiment qui contient une composition (B) anhydre comprenant au moins un sel peroxygéné et au moins un agent alcalin tels que définis précédemment, et un troisième compartiment qui contient une composition (C) aqueuse de peroxyde d'hydrogène.

Selon un autre mode de réalisation particulier de l'invention, le dispositif conforme à invention comporte un premier compartiment qui contient une composition (D) comprenant, dans un milieu approprié pour la teinture, au moins un colorant anionique ou non ionique tel que défini précédemment, un deuxième compartiment qui contient une composition (E) anhydre comprenant au moins un liquide inerte organique, au moins un sel peroxygéné et au moins un agent alcalin tels que définis précédemment, et un troisième compartiment qui contient une composition (C) aqueuse de peroxyde d'hydrogène.

Selon un autre mode de réalisation particulier de l'invention, le dispositif conforme à invention comporte un premier compartiment qui contient une composition (F) anhydre comprenant au moins un colorant anionique ou non ionique, au moins un liquide inerte organique, au moins un sel peroxygéné et au moins un agent alcalin tels que définis précédemment, et un deuxième compartiment qui contient une composition (C) aqueuse de peroxyde d'hydrogène.

Selon un autre mode de réalisation particulier de l'invention, le dispositif conforme à invention comporte un premier compartiment qui contient une composition (E) anhydre comprenant au moins un liquide inerte organique, au moins un sel peroxygéné et au moins un agent alcalin tels que définis précédemment, et un deuxième compartiment qui contient une composition (G) comprenant, dans un milieu approprié pour la teinture, au moins un colorant anionique ou non ionique tel que défini précédemment et du peroxyde d'hydrogène.

Selon un autre mode de réalisation particulier de l'invention, le dispositif conforme à invention comporte un premier compartiment qui contient une composition (B) anhydre comprenant au moins un sel peroxygéné et au moins un agent alcalin tels que définis précédemment, et un deuxième compartiment qui contient une composition (H) comprenant, dans un milieu approprié pour la teinture, au moins un colorant anionique ou non ionique et au moins un liquide inerte organique tels que définis précédemment et du peroxyde d'hydrogène.

Le milieu approprié pour la teinture des compositions (A), (C), (D), (G) et (H) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids par rapport au poids total de la composition tinctoriale, et plus préférentiellement encore entre 5 et 30 % en poids environ.

Les compositions (A) et (D), encore appelée "booster", peuvent être formulées à pH acide, neutre ou alcalin, le pH pouvant varier entre 3 et 12 environ et de préférence entre 4 et 11 environ.

Les compositions (C), (G) et (H) présentent de préférence un pH inférieur à 7, le pH acide garantissant la stabilité du peroxyde d'hydrogène dans cette composition.

Les compositions (A), (C), (D), (G) et (H) peuvent se présenter sous des formes diverses, telles que sous forme de liquides, de crèmes, de gels, ou sous toute autre forme appropriée pour réaliser une teinture des fibres kératiniques.

Les compositions (E) et (F) anhydres se présentent sous forme de pâte.

La composition (B) anhydre peut se présenter sous forme de poudre ou de pâte. Dans le cas où elle se présente sous forme de pâte, elle comprend de plus un liquide inerte organique tel que défini précédemment.

Les compositions (A) à (I) peuvent également renfermer divers additifs classiquement utilisés en cosmétique tels que ceux qui sont décrits précédemment.

Les compositions (C), (G) et (H) peuvent de plus comprendre des agents de contrôle du dégagement d'oxygène tels que définis précédemment.

Le dispositif conforme à la présente invention peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de décolorer et de colorer simultanément les fibres kératiniques à partir d'un procédé conforme à l'invention tel que défini précédemment.

La présente invention a aussi pour objet l'utilisation pour la décoloration et la coloration simultanées des fibres kératiniques d'une composition conforme à l'invention telle que définie précédemment.

Les exemples qui suivent servent à illustrer l'invention sans toutefois présenter un caractère limitatif.

### EXEMPLES

On a préparé les compositions suivantes :

| **Composition (pâte décolorante)** | **A** | **B** | **C** |
|---|---|---|---|
| Isopropyl myristate | 20 g | 20 g | 20 g |
| Gomme xanthane | 1,4 g | 1,4 g | 1,4 g |
| Ultramarines | 0,5 g | 0,5 g | 0,5 g |
| Oxyde de magnésium | 2,0 g | 2,0 g | 2,0 g |
| Silicate de sodium | 15,0 g | 15,0 g | 15,0 g |
| Dioxyde de titane | 1,0 g | 1,0 g | 1,0 g |
| 2-oléamido-1,3-octadécanediol | 0,01 g | 0,01 g | 0,01 g |
| Stéarate de magnésium | 2,0 g | 2,0 g | 2,0 g |
| EDTA | 0,2 g | 0,2 g | 0,2 g |
| Lauryl sulfate de sodium | 4,0 g | 4,0 g | 4,0 g |
| Silice | 2,5 g | 2,5 g | 2,5 g |
| Persulfate de potassium | 39,08 g | 39,08 g | 39,08 g |
| Persulfate de sodium | 6,0 g | 6,0 g | 6,0 g |
| Cire d'abeille | 1,2 g | 1,2 g | 1,2 g |
| Huile de vaseline | 1,0 g | 1,0 g | 1,0 g |
| Sel de sodium du carboxyméthyl amidon | 2,5 g | 2,5 g | 2,5 g |
| Hydroxyéthyl-2-nitro-para-toluidine | 1,6 g | - | - |
| 3-méthylamino-4-nitrophénoxyéthanol | - | 1,6 g | - |
| Acid Red 87 | - | - | 1,6 g |

| **Composition oxydante** | |
|---|---|
| Stannate de sodium | 0,04 g |
| Pentétate de pentasodium | 0,06 g |
| Alcool cétéarylique | 8,00 g |
| Ceteareth-33 | 2,00 g |
| Acide 2-phosphorique | q.s.p. pH = 3 |
| Peroxyde d'hydrogène | 9,00 g |
| Pyrophosphate de tétrasodium | 0,03 g |
| Eau | 80,87 g |

Les compositions A, B et C sont mélangées au moment de l'emploi à la composition oxydante avec un rapport pâte décolorante / composition oxydante égal à 1 / 1,5.

Les mélanges ainsi obtenus sont appliqués immédiatement et de manière différée sur une mèche de cheveux gris à 90 % de cheveux blancs naturels et sur une mèche de cheveux châtains naturels, à raison de 10 g de mélange pour 1 g de cheveux. Après un temps de pose de 30 minutes, les mèches sont rincées, lavées avec un shampooing standard, rincées à nouveau puis séchées.

Les reflets obtenus sont décrits dans le tableau ci-dessous.

| **Composition A** | cheveux gris à 90 % de cheveux blancs naturels | cheveux châtains naturels |
|---|---|---|
| Application immédiate | Jaune cuivré profond | Cuivré doré |
| Application différée | Jaune cuivré profond | Cuivré doré |

| **Composition B** | cheveux gris à 90 % de cheveux blancs naturels | cheveux châtains naturels |
|---|---|---|
| Application immédiate | Jaune vif | Doré |
| Application différée | Jaune vif | Doré |

| **Composition C** | cheveux gris à 90 % de cheveux blancs naturels | cheveux châtains naturels |
|---|---|---|
| Application immédiate | Rose | Cuivré orangé |
| Application différée | Rose | Cuivré orangé |

On constate que les reflets obtenus sont les mêmes lorsque l'application est immédiate et lorsqu'elle est différée, ce qui montre que les colorants sont stables dans les compositions conformes à l'invention en présence de peroxyde d'hydrogène.

## Revendications

1. Composition pour la décoloration et la coloration simultanées des fibres kératiniques comprenant :
- au moins un colorant choisi parmi les colorants anioniques et non ioniques, à l'exception du 7-(6'-méthylphénylazo)-1-acétamido-3,6-disulfo-8-hydroxy-naphtalène, des ortho nitro-anilines substituées en méta du groupement amino, de la quinoline, des dérivés quinoliniques, et de leurs sels d'addition ;
- au moins un liquide inerte organique ;
- au moins un sel peroxygéné ; et
- au moins un agent alcalin.

2. Composition selon la revendication 1, dans laquelle le ou les colorants anioniques sont choisis parmi les colorants directs nitrés acides, les colorants azoïques acides, les colorants aziniques acides, les colorants triarylméthaniques acides, les colorants indoaminiques acides, les colorants naturels acides.

3. Composition selon la revendication 2, dans laquelle le ou les colorants anioniques sont choisis parmi les composés suivants :
| | |
|---|---|
| (C.I. 45380) | Acid Red 87 |
| (C.I. 10316) | Sel de sodium de l'acide 2,4-dinitro-1-naphtol-7-sulfonique |
| (C.I. 10383) | Acid Orange 3 |
| (C.I. 13015) | Acid Yellow 9 / Food Yellow 2 |
| (C.I. 14780) | Direct Red 45 / Food Red 13 |
| (C.I. 13711) | Acid Black 52 |
| (C.I. 13065) | Acid Yellow 36 |
| (C.I. 14700) | Sel de sodium de l'acide 1-hydroxy-2-(2',4'-xylyl-5-sufonatoazo)-naphtalène-4-sulfonique / Food Red 1 |
| (C.I. 14720) | Acid Red 14 / Food Red 3 / Mordant Blue 79 |
| (C. I. 14805) | Acid Brown 4 |
| (C.I. 15510) | Acid Orange 7 / Pigment Orange 17 / Solvent Orange 49 |
| (C.I. 15985) | Food Yellow 3 / Pigment Yellow 104 |
| (C.I. 16185) | Acid Red 27 / Food Red 9 |
| (C.I. 16230) | Acid Orange 10 / Food Orange 4 |
| (C.I. 16250) | Acid Red 44 |
| (C.I. 17200) | Acid Red 33 / Food Red 12 |
| (C.I. 15685) | Acid Red 184 |
| (C.I. 19125) | Acid Violet 3 |
| (C.I. 18055) | Sel de sodium de l'acide 1-hydroxy-2-(4'-acétamido phénylazo)-8-acétamido-naphtalène-3,6-disulfonique / Acid Violet 7 / Food Red 11 |
| (C.I. 18130) | Acid Red 135 |
| (C.I. 19130) | Acid Yellow 27 |
| (C.I. 19140) | Acid Yellow 23 / Food Yellow 4 |
| (C.I. 20170) | 4'-(sulfonato-2",4"-diméthyl)-bis-(2,6-phénylazo)-1,3-dihydroxy benzène / Acid Orange 24 |
| (C.I. 20470) | Sel de sodium de l'acide 1-amino-2-(4'-nitrophénylazo)-7-phénylazo-8-hydroxy-naphtalène-3,6-disulfonique / Acid Black 1 |
| (C.I. 23266) | (4-((4-méthylphényl) sulfonyloxy)-phénylazo)2,2'-diméthyl-4-((2-hydroxy-5,8-disulfonato)naphtylazo)biphényle / Acid Red 111 |
| (C.I. 27755) | Food Black 2 |
| (C.I. 25440) | 1-(4'-sulfonatophénylazo)-4-((2"-hydroxy-3"-acétylamino-6",8"-disulfonato)naphtylazo)-6-sulfonatonaphtalène (sel tétrasodique) / Food Black 1 |
| (C.I. 42080) | Acide 4-β-hydroxyéthylamino-3-nitrobenzènesulfonique |
| (C.I. 42090) | Acid Blue 9 |
| (C.I. 60730) | Acid Violet 43 |
| (C.I. 61570) | Acid Green 25 |
| (C.I. 62045) | Sel de sodium de l'acide 1-amino-4-cyclohexylamino-9,10-anthraquinone 2-sulfonique / Acid Blue 62 |
| (C.I. 62105) | Acid Blue 78 |
| (C.I. 14710) | Sel de sodium de l'acide 4-hydroxy-3((2-méthoxy phényl)-azo)-1-naphtalène sulfonique / Acid Red 4 |
| | Acide 2-pipéridino 5-nitro benzène sulfonique |
| | Acide 2(4'-N,N(2"-hydroxyéthyl)amino-2'-nitro)aniline éthane sulfonique |
| | Acide 4-β-hydroxyéthylamino-3-nitrobenzène sulfonique |
| (C.I. 42640) | Acid Violet 49 |
| (C.I. 42080) | Acid Blue 7 |
| | Acid Blue 156 |
| | Acid Blue 317 |
| (C.I. 58005) | Sel de sodium du 1,2-dihydroxy-3-sulfo-anthraquinone / Mordant Red 3 |
| (C.I. 62055) | Sel de sodium de l'acide 1-amino-9,10-dihydro-9,10-dioxo-4-(phénylamino) 2-anthracène sulfonique / Acid Blue 25 |
| (C.I. 14710) | Sel de sodium de l'acide 4-hydroxy-3-((2-méthoxyphényl)-azo)-1-naphtalène sulfonique / Acid Red 4 |

4. Composition selon l'une quelconque des revendications précédentes, dans laquelle le ou les colorants non ioniques sont choisis parmi les colorants non ioniques benzéniques nitrés, les colorants non ioniques azoïques, azométhiniques, méthiniques, anthraquinoniques, naphtoquinoniques, benzoquinoniques, phénotiaziniques indigoïdes, xanthéniques, phénanthridiniques, phtalocyanines, ceux dérivés du triarylméthane, seuls ou en mélanges.

5. Composition selon la revendication 4, dans laquelle le ou les colorants non ioniques sont choisis parmi le 1-hydroxy-3-nitro-4-N-(y-hydroxypropyl)amino benzène ; le N-(β-hydroxyéthyl)amino-3-nitro-4-amino benzène; le 1-hydroxy-3-nitro-4-N-(β-hydroxyéthyl)amino benzène; le 1,4-diamino-2-nitrobenzène ; le 1-amino-2-nitro-4-méthylamino benzène ; la N-(β-hydroxyéthyl)-2-nitro-paraphénylènediamine ; la 2-nitro-4-amino-diphénylamine ; le 1-amino-3-nitro-6-hydroxybenzène ; le 1-(β-aminoéthyl)amino-2-nitro-4-(β-hydroxyéthyloxy) benzène ; le 1-(β, γ-dihydroxypropyl)oxy-3-nitro-4-(β-hydroxyéthyl)amino benzène ; le 1-hydroxy-3-nitro-4-aminobenzène ; le 1-hydroxy-2-amino-4,6-dinitrobenzène ; le 1-méthoxy-3-nitro-4-(β-hydroxyéthyl)amino benzène ; la 2-nitro-4'-hydroxydiphénylamine ; le 1-(β-hydroxyéthyl)amino-2-méthoxy-4-nitrobenzène ; le 1-amino-2-nitro-6-méthyl-benzène ; le 1-(β-hydroxyéthyl)amino-2-hydroxy-4-nitrobenzène ; la N-(β-hydroxyéthyl)-2-nitro-4-trifluorométhylaniline ; le 4-(β-hydroxyéthyl)amino-3-nitro-chlorobenzène ; le 4-(β-hydroxyéthyl)amino-3-nitro-méthylbenzène ; le 4-(β,γ -dihydroxypropyl)amino-3-nitro-trifluorométhylbenzène ; le 1-(β-uréidoéthyl)amino-4-nitrobenzène; le 1-hydroxy-2-amino-5-nitrobenzène ; le 1-amino-2-[tris(hydroxyméthyl)méthyl]amino-5-nitro-benzène ; le 1-(β-hydroxyéthyl)amino-2-nitrobenzène ; le 4-(β-hydroxyéthyl)amino-3-nitrobenzamide ; le 1-(β-hydroxyéthyl)amino-4-N,N-bis-(β-hydroxyéthyl)amino 2-nitrobenzène ; le 1-(y-hydroxypropyl)amino 4-N,N-bis-(β-hydroxyéthyl)amino 2-nitrobenzène ; le 1-(β-hydroxyéthyl)amino 4-(N-méthyl, N-β-hydroxyéthyl)amino 2-nitrobenzène ; le 1-(β-hydroxyéthyl)amino 4-(N-éthyl, N-β-hydroxyéthyl)amino 2-nitrobenzène ; le 1-(β,γ-dihydroxypropyl)amino 4-(N-éthyl, N-β-hydroxyéthyl)amino 2-nitrobenzène ; le Disperse Orange 3 ; le Disperse Red 17 ; le Disperse Violet 4 ; le Disperse Violet 8 ; le Disperse Blue 1 ; le Disperse Red 15 ; le Solvent Violet 13 ; le Solvent Violet 11 ; le Disperse Blue 3 ; le Disperse Blue 7 ; le Disperse Red 11 ; le Natural Brown 7 ; le Disperse Black 9 ; le Disperse Violet 15 ; le Natural Orange 6 ; la 2-hydroxy 3-méthoxy 1,4-naphtoquinone ; la 3-hydroxy 2-méthyl 1,4-naphtoquinone ou le phtiocol ; la 3,6-dihydroxy 5-méthoxy p-toluquinone ou la spinulosine ; le HC Blue 14.

6. Composition selon l'une quelconque des revendications précédentes, dans laquelle la concentration en colorants anioniques et / ou non ioniques est comprise entre 0,0001 et 10 % en poids du poids total de la composition.

7. Composition selon l'une quelconque des revendications précédentes, dans laquelle le ou les liquides inertes organiques sont choisies parmi par les polydécènes de formule C₁₀ₙH_{[(20n)+2]} dans laquelle n varie de 3 à 9, les esters d'alcools gras ou d'acides gras, les esters ou di-esters de sucres d'acides gras en C₁₂-C₂₄, les éthers cycliques ou les esters cycliques, les huiles de silicone, les huiles minérales, les huiles végétales, et leurs mélanges.

8. Composition selon la revendication 7, dans laquelle le ou les liquides inertes organiques sont choisies parmi les polydécènes de formule C₁₀ₙH_{[(20n)+2]} dans laquelle n varie de 3 à 9, les esters d'alcools gras ou d'acides gras, et leurs mélanges.

9. Composition selon l'une quelconque des revendications précédentes, dans laquelle la concentration en liquides inertes organiques est comprise entre 5 et 60 % en poids du poids total de la composition.

10. Composition selon l'une quelconque des revendications précédentes, dans laquelle le ou les sels peroxygénés sont choisis parmi les persulfates, les perborates, les percarbonates, les peroxydes de métaux alcalins ou alcalino-terreux, et leurs mélanges.

11. Composition selon la revendication 10, dans laquelle le ou les sels peroxygénés sont choisis parmi les persulfates et leurs mélanges.

12. Composition selon la revendication 11, dans laquelle le ou les sels peroxygénés sont choisis parmi le persulfate de sodium, le persulfate de potassium, le persulfate d'ammonium, et leurs mélanges.

13. Composition selon l'une quelconque des revendications précédentes, dans laquelle la concentration en sels peroxygénés est comprise entre 1 et 70 % en poids du poids total de la composition.

14. Composition selon l'une quelconque des revendications précédentes, dans laquelle le ou les agents alcalins sont choisis parmi l'urée, le chlorure d'ammonium, le sulfate d'ammonium, le phosphate d'ammonium, le nitrate d'ammonium, les silicates, les phosphates ou les carbonates de métaux alcalins ou alcalino-terreux, et leurs mélanges.

15. Composition selon l'une quelconque des revendications précédentes, dans laquelle la concentration en agents alcalins est comprise entre 0,01 et 40 % en poids du poids total de la composition.

16. Composition selon l'une quelconque des revendications précédentes, **caractérisée en ce qu'**elle est anhydre.

17. Composition selon l'une quelconque des revendications 1 à 15, **caractérisée en ce qu'**elle est aqueuse.

18. Composition selon la revendication 17, comprenant de plus du peroxyde d'hydrogène.

19. Procédé de décoloration et de coloration simultanées des fibres kératiniques, **caractérisé par le fait que** l'on applique sur lesdites fibres kératiniques une composition anhydre telle que définie à la revendication 16 en présence d'une composition aqueuse comprenant ou non du peroxyde d'hydrogène.

20. Dispositif à plusieurs compartiments, **caractérisé par le fait qu'**il contient au moins deux compositions dont le mélange conduit à une composition aqueuse telle que définie à la revendication 17 ou 18.

21. Utilisation pour la décoloration et la coloration simultanées d'une composition telle que définie à l'une quelconque des revendications 1 à 18.

## Claims

1. Composition for simultaneously bleaching and dyeing keratin fibres, comprising:
- at least one dye chosen from anionic and nonionic dyes, with the exception of 7-(6'-methylphenylazo)-1-acetamido-3,6-disulfo-8-hydroxynaphthalene, ortho-nitroanilines substituted meta to the amino group, quinoline and quinoline derivatives, and addition salts thereof;
- at least one inert organic liquid;
- at least one peroxygenated salt; and
- at least one alkaline agent.

2. Composition according to Claim 1, in which the anionic dye(s) is (are) chosen from acidic nitro direct dyes, acidic azo dyes, acidic azine dyes, acidic triarylmethane dyes, acidic indoamine dyes and acidic natural dyes.

3. Composition according to Claim 2, in which the anionic dye(s) is (are) chosen from the following compounds:
| | |
|---|---|
| (C.I. 45380) | Acid Red 87 |
| (C.I. 10316) | Sodium salt of 2,4-dinitro-1-naphthol-7-sulfonic acid |
| (C.I. 10383) | Acid Orange 3 |
| (C.I. 13015) | Acid Yellow 9/Food Yellow 2 |
| (C.I. 14780) | Direct Red 45/Food Red 13 |
| (C.I. 13711) | Acid Black 52 |
| (C.I. 13065) | Acid Yellow 36 |
| (C.I. 14700) | Sodium salt of 1-hydroxy-2- (2',4'-xylyl-5-sulfonatoazo)naphthalene-4-sulfonic acid/Food Red 1 |
| (C.I. 14720) | Acid Red 14/Food Red 3/Mordant Blue 79 |
| (C.I. 14805) | Acid Brown 4 |
| (C.I. 15510) | Acid Orange 7/Pigment Orange 17/Solvent Orange 49 |
| (C.I. 15985) | Food Yellow 3/Pigment Yellow 104 |
| (C.I. 16185) | Acid Red 27/Food Red 9 |
| (C.I. 16230) | Acid Orange 10/Food Orange 4 |
| (C.I. 16250) | Acid Red 44 |
| (C.I. 17200) | Acid Red 33/Food Red 12 |
| (C.I. 15685) | Acid Red 184 |
| (C.I. 19125) | Acid Violet 3 |
| (C.I. 18055) | Sodium salt of 1-hydroxy-2-(4-acetamidophenylazo)-8-acetamidonaphthalene-3,6-disulfonic acid/Acid Violet 7/Food Red 11 |
| (C.I. 18130) | Acid Red 135 |
| (C.I. 19130) | Acid Yellow 27 |
| (C.I. 19140) | Acid Yellow 23/Food Yellow 4 |
| (C.I. 20170) | 4'-(Sulfonato-2",4"-dimethyl)bis(2,6-phenylazo)-1,3-dihydroxybenzene/Acid Orange 24 |
| (C.I. 20470) | Sodium salt of 1-amino-2-(4'-nitrophenylazo)-7-phenylazo-8-hydroxynaphthalene-3,6-disulfonic acid/Acid Black 1 |
| (C.I. 23266) | (4-((4-Methylphenyl)sulfonyloxy)phenylazo)-2,2'-dimethyl-4-((2-hydroxy-5,8-disulfonato)naphthylazo)biphenyl/Acid Red 111 |
| (C.I. 27755) | Food Black 2 |
| (C.I. 25440) | 1-(4'-Sulfonatophenylazo)-4-((2"-hydroxy3"-acetylamino-6",8"-disulfonato)naphthylazo)-6-sulfonatonaphthalene (tetrasodium salt)/Food Black 1 |
| (C.I. 42080) | 4-β-Hydroxyethylamino-3-nitrobenzenesulfonic acid |
| (C.I. 42090) | Acid Blue 9 |
| (C.I. 60730) | Acid Violet 43 |
| (C.I. 61570) | Acid Green 25 |
| (C.I. 62045) | Sodium salt of 1-amino-4-cyclohexylamino-9, 10-anthraquinone-2-sulfonic acid/Acid Blue 62 |
| (C.I. 62105) | Acid Blue 78 |
| (C.I. 14710) | Sodium salt of 4-hydroxy-3-((2-methoxyphenyl)azo)-1-naphthalenesulfonic acid/Acid Red 4 |
| | 2-Piperidino-5-nitrobenzenesulfonic acid |
| | 2-(4'-N,N-(2"-Hydroxyethyl)amino-2'-nitro)-anilinethanesulfonic acid |
| | 4-β-Hydroxyethylamino-3-nitrobenzenesulfonic acid |
| (C.I. 42640) | Acid Violet 49 |
| (C.I. 42080) | Acid Blue 7 |
| | Acid Blue 156 |
| | Acid Blue 317 |
| (C.I. 58005) | Sodium salt of 1,2-dihydroxy-3-sulfoanthraquinone/Mordant Red 3 |
| (C.I. 62055) | Sodium salt of 1-amino-9,10-dihydro-9,10-dioxo-4-(phenylamino)-2-anthracenesulfonic acid/Acid Blue 25 |
| (C.I. 14710) | Sodium salt of 4-hydroxy-3-((2-methoxyphenyl)azo)-1-naphthalenesulfonic acid/Acid Red 4 |

4. Composition according to any one of the preceding claims, in which the nonionic dye(s) is (are) chosen from nonionic nitrobenzene dyes and nonionic azo, azomethine, methine, anthraquinone, naphthoquinone, benzoquinone, phenothiazine, indigoid, xanthene, phenanthridine, phthalocyanin and triarylmethane-based dyes, alone or as mixtures.

5. Composition according to Claim 4, in which the nonionic dye(s) is (are) chosen from 1-hydroxy-3-nitro-4-N-(γ-hydroxypropyl)aminobenzene; N-(β-hydroxyethyl)-amino-3-nitro-4-aminobenzene; 1-hydroxy-3-nitro-4-N-(β-hydroxyethyl)aminobenzene; 1,4-diamino-2-nitrobenzene; 1-amino-2-nitro-4-methylaminobenzene; N-(β-hydroxyethyl)-2-nitro-para-phenylenediamine; 2-nitro-4-amino-diphenylamine; 1-amino-3-nitro-6-hydroxybenzene; 1-(β-aminoethyl)amino-2-nitro-4-(β-hydroxyethyloxy)benzene; 1-(β,γ-dihydroxypropyl)oxy-3-nitro-4-(β-hydroxyethyl)-aminobenzene; 1-hydroxy-3-nitro-4-aminobenzene; 1-hydroxy-2-amino-4,6-dinitrobenzene; 1-methoxy-3-nitro-4-(β-hydroxyethyl)aminobenzene; 2-nitro-4'-hydroxydiphenylamine; 1-(β-hydroxyethyl)amino-2-methoxy-4-nitrobenzene; 1-amino-2-nitro-6-methylbenzene; 1-(β-hydroxyethyl)amino-2-hydroxy-4-nitrobenzene; N-(β-hydroxyethyl)-2-nitro-4-trifluoromethylaniline; 4-(β-hydroxyethyl)amino-3-nitrochlorobenzene; 4-(β-hydroxyethyl)amino-3-nitromethylbenzene; 4-(β,γ-dihydroxypropyl)amino-3-nitrotrifluoromethylbenzene; 1-(β-ureidoethyl)amino-4-nitrobenzene; 1-hydroxy-2-amino-5-nitrobenzene; 1-amino-2-[tris(hydroxymethyl)methyl]amino-5-nitrobenzene; 1-(β-hydroxyethyl)amino-2-nitrobenzene; 4-(β-hydroxyethyl)amino-3-nitrobenzamide; 1-(β-hydroxyethyl)amino-4-N,N-bis(β-hydroxyethyl)amino-2-nitro-benzene; 1-(γ-hydroxypropyl)amino-4-N,N-bis(β-hydroxyethyl)amino-2-nitrobenzene; 1-(β-hydroxyethyl)amino-4-(N-methyl-N-β-hydroxyethyl)amino-2-nitrobenzene; 1-(β-hydroxyethyl)amino-4-(N-ethyl-N-β-hydroxyethyl)amino-2-nitrobenzene; 1-(β,γ-dihydroxypropyl)amino-4-(N-ethyl-N-β-hydroxyethyl)amino-2-nitrobenzene; Disperse Orange 3; Disperse Red 17; Disperse Violet 4; Disperse Violet 8; Disperse Blue 1; Disperse Red 15; Solvent Violet 13; Solvent Violet 11; Disperse Blue 3; Disperse Blue 7; Disperse Red 11; Natural Brown 7; Disperse Black 9; Disperse Violet 15; Natural Orange 6; 2-hydroxy-3-methoxy-1,4-naphthoquinone; 3-hydroxy-2-methyl-1,4-naphthoquinone or phthiocol; 3,6-dihydroxy-5-methoxy-p-toluquinone or spinulosin; HC Blue 14.

6. Composition according to any one of the preceding claims, in which the concentration of anionic and/or nonionic dyes is between 0.0001% and 10% by weight relative to the total weight of the composition.

7. Composition according to any one of the preceding claims, in which the inert organic liquid(s) is (are) chosen from the polydecenes of formula C₁₀ₙH_{[(20n)+2]} in which n ranges from 3 to 9, esters of fatty alcohols or of fatty acids, sugar esters or diesters of C₁₂-C₂₄ fatty acids, cyclic ethers or cyclic esters, silicone oils, mineral oils and plant oils, and mixtures thereof.

8. Composition according to Claim 7, in which the inert organic liquid(s) is (are) chosen from the polydecenes of formula C₁₀ₙH_{[(20n)+2]} in which n ranges from 3 to 9, and esters of fatty alcohols or of fatty acids, and mixtures thereof.

9. Composition according to any one of the preceding claims, in which the concentration of inert organic liquids is between 5% and 60% by weight relative to the total weight of the composition.

10. Composition according to any one of the preceding claims, in which the peroxygenated salt(s) is (are) chosen from alkali metal or alkaline-earth metal persulfates, perborates, percarbonates and peroxides, and mixtures thereof.

11. Composition according to Claim 10, in which the peroxygenated salt(s) is (are) chosen from persulfates and mixtures thereof.

12. Composition according to Claim 11, in which the peroxygenated salt(s) is (are) chosen from sodium persulfate, potassium persulfate and ammonium persulfate, and mixtures thereof.

13. Composition according to any one of the preceding claims, in which the concentration of peroxygenated salts is between 1% and 70% by weight relative to the total weight of the composition.

14. Composition according to any one of the preceding claims, in which the alkaline agent(s) is (are) chosen from urea, ammonium chloride, ammonium sulfate, ammonium phosphate, ammonium nitrate, and alkali metal or alkaline-earth metal silicates, phosphates or carbonates, and mixtures thereof.

15. Composition according to any one of the preceding claims, in which the concentration of alkaline agents is between 0.01% and 40% by weight relative to the total weight of the composition.

16. Composition according to any one of the preceding claims, **characterized in that** it is anhydrous.

17. Composition according to any one of Claims 1 to 15, **characterized in that** it is aqueous.

18. Composition according to Claim 17, also comprising hydrogen peroxide.

19. Process for simultaneously bleaching and dyeing keratin fibres, **characterized in that** an anhydrous composition as defined in Claim 16 is applied to the said keratin fibres in the presence of an aqueous composition optionally comprising hydrogen peroxide.

20. Multi-compartment device, **characterized in that** it contains at least two compositions, the mixing of which leads to an aqueous composition as defined in Claim 17 or 18.

21. Use, for simultaneously bleaching and dyeing, of a composition as defined in any one of Claims 1 to 18.

## Patentansprüche

1. Zusammensetzung zum Entfärben und gleichzeitigen Färben von Keratinfasern, die enthält:
- mindestens einen Farbstoff, der unter den anionischen und nichtionischen Farbstoffen mit Ausnahme von 7-(6'-Methylphenylazo)-1-acetamido-3,6-disulfo-8-hydroxynaphthalin, in meta-Stellung der Aminogruppe substituierten ortho-Nitroanilinen, Chinolin, Chinolinderivaten und deren Additionssalzen ausgewählt ist;
- mindestens eine inerte organische Flüssigkeit;
- mindestens ein Peroxysalz; und
- mindestens eine Base.

2. Zusammensetzung nach Anspruch 1, bei der der oder die anionische(n) Farbstoff(e) unter den sauren nitrierten Direktfarbstoffen, sauren Azofarbstoffen, sauren Azinfarbstoffen, sauren Triarylmethan-Farbstoffen, sauren Indoamin-Farbstoffen und sauren natürlichen Farbstoffen ausgewählt sind.

3. Zusammensetzung nach Anspruch 2, bei der der oder die anionische(n) Farbstoff(e) unter den folgenden Verbindungen ausgewählt sind:
| | |
|---|---|
| (C.I. 45380) | Acid Red 87 |
| (C.I. 10316) | Natriumsalz der 2,4-Dinitro-1-naphthol-7-sulfonsäure |
| (C.I. 10383) | Acid Orange 3 |
| (C.I. 13015) | Acid Yellow 9 / Food Yellow 2 |
| (C.I. 14780) | Direct Red 45 / Food Red 13 |
| (C.I. 13711) | Acid Black 52 |
| (C.I. 13065) | Acid Yellow 36 |
| (C.I. 14700) | Natriumsalz der 1-Hydroxy-2-(2',4'-xylyl-5-sufonatoazo)-naphthalin-4-sulfonsäure/Food Red 1 |
| (C.I. 14720) | Acid Red 14/Food Red 3/Mordant Blue 79 |
| (C. I. 14805) | Acid Brown 4 |
| (C.I. 15510) | Acid Orange 7/Pigment Orange 17/Solvent Orange 49 |
| (C.I. 15985) | Food Yellow 3 / Pigment Yellow 104 |
| (C.I. 16185) | Acid Red 27 / Food Red 9 |
| (C.I. 16230) | Acid Orange 10 / Food Orange 4 |
| (C.I. 16250) | Acid Red 44 |
| (C.I. 17200) | Acid Red 33 / Food Red 12 |
| (C.I. 15685) | Acid Red 184 |
| (C.I. 19125) | Acid Violet 3 |
| (C.I. 18055) | Natriumsalz der 1-Hydroxy-2-(4'-acetamidophenylazo)-8-acetamido-naphthalin-3,6-disulfonsäure/Acid Violet 7 / Food Red 11 |
| (C.I. 18130) | Acid Red 135 |
| (C.I. 19130) | Acid Yellow 27 |
| (C.1. 19140) | Acid Yellow 23/Food Yellow 4 |
| (C.I. 20170) | 4'-(Sulfonato-2",4"-dimethyl)-bis-(2,6-phenylazo)-1,3-dihydroxy-benzol/Acid Orange 24 |
| (C.I. 20470) | Natriumsalz der 1-Amino-2-(4'-nitrophenylazo)-7-phenylazo-8-hydroxy-naphthalin-3,6-disulfonsäure/Acid Black 1 |
| (C.I. 23266) | (4-((4-Methylphenyl)sulfonyloxy)-phenylazo)-2,2'-dimethyl-4-((2-hydroxy-5,8-disulfonato)-naphthylazo)biphenyl/Acid Red 111 |
| (C.I. 27755) | Food Black 2 |
| (C.I. 25440) | 1-(4'-Sulfonatophenylazo)-4-((2"-hydroxy-3"-acetylamino-6",8"-disulfonato)naphtylazo)-6-sulfonatonaphthalin (Tetranatriumsalz)/Food Black 1 |
| (C.I. 42080) | 4-β-Hydroxyethylamino-3-nitrobenzolsulfonsäure |
| (C.I. 42090) | Acid Blue 9 |
| (C.I. 60730) | Acid Violet 43 |
| (C.I. 61570) | Acid Green 25 |
| (C.I. 62045) | Natriumsalz der 1-Amino-4-cyclohexylamino-9,10-anthrachinon-2-sulfonsäure/Acid Blue 62 |
| (C.I. 62105) | Acid Blue 78 |
| (C.I. 14710) | Natriumsalz der 4-Hydroxy-3-((2-methoxyphenyl)-azo)-1-naphthalin-sulfonsäure/Acid Red 4 |
| | 2-Piperidino-5-nitro-benzolsulfonsäure |
| | 2-(4'-N,N-(2"-Hydroxyethyl)amino-2'-nitro)-anilin-ethan-sulfonsäure |
| | 4-β-Hydroxyethylamino-3-nitrobenzol-sulfonsäure |
| (C.I. 42640) | Acid Violet 49 |
| (C.I. 42080) | Acid Blue 7 |
| | Acid Blue 156 |
| | Acid Blue 317 |
| (C.I. 58005) | Natriumsalz von 1,2-Dihydroxy-3-sulfoanthrachinon/Mordant Red 3 |
| (C.I. 62055) | Natriumsalz der 1-Amino-9,10-dihydro-9,10-dioxo-4-(phenylamino)-2-anthracen-sulfonsäure/Acid Blue 25 |
| (C.I. 14710) | Natriumsalz der 4-Hydroxy-3-((2-methoxyphenyl)-azo)-1-naphthalin-sulfonsäure/Acid Red 4 |

4. Zusammensetzung nach einem der vorhergehenden Ansprüche, bei der der oder die nichtionische(n) Farbstoff(e) unter den nichtionischen nitrierten Benzolfarbstoffen, nichtionischen Azofarbstoffen, Azomethin-Farbstoffen, Methin-Farbstoffen, Anthrachinon-Farbstoffen, Naphthochinon-Farbstoffen, Benzochinon-Farbstoffen, Phenothiazinindigoiden, Xanthen-Farbstoffen, Phenanthridin-Farbstoffen, Phthalocyaninen, von Triarylmethan abgeleiteten Farbstoffen oder deren Gemischen ausgewählt sind.

5. Zusammensetzung nach Anspruch 4, bei der der oder die nichtionische(n) Farbstoff(e) unter den folgenden Verbindungen ausgewählt sind: 1-Hydroxy-3-nitro-4-N-(y-hydroxypropyl)aminobenzol; N-(β-Hydroxyethyl)amino-3-nitro-4-amino-benzol; 1-Hydroxy-3-nitro-4-N-(β-hydroxyethyl)amino-benzol; 1,4-Diamino-2-nitro-benzol; 1-Amino-2-nitro-4-methylamino-benzol; N-(β-Hydroxyethyl)-2-nitro-paraphenylendiamin; 2-Nitro-4-amino-diphenylamin; 1-Amino-3-nitro-6-hydroxy-benzol; 1-(β-Aminoethyl)amino-2-nitro-4-(β-hydroxyethyloxy)-benzol; 1-(β,γ-Dihydroxypropyl)oxy-3-nitro-4-(β-hydroxyethyl)amino-benzol; 1-Hydroxy-3-nitro-4-amino-benzol; 1-Hydroxy-2-amino-4,6-dinitro-benzol; 1-Methoxy-3-nitro-4-(β-hydroxyethyl)amino-benzol; 2-Nitro-4'-hydroxydiphenylamin; 1-(β-Hydroxyethyl)amino-2-methoxy-4-nitro-benzol; 1-Amino-2-nitro-6-methyl-benzol; 1-(β-Hydroxyethyl)amino-2-hydroxy-4-nitro-benzol; N-(β-Hydroxyethyl)-2-nitro-4-trifluormethylanilin; 4-(β-Hydroxyethyl)amino-3-nitro-chlor-benzol; 4-(β-Hydroxyethyl)amino-3-nitro-methylbenzol; 4-(β,γ-Dihydroxypropyl)amino-3-nitro-trifluormethyl-benzol; 1-(β-Ureidoethyl)amino-4-nitro-benzol; 1-Hydroxy-2-amino-5-nitro-benzol; 1-Amino-2-[tris(hydroxymethyl)methyl]-amino-5-nitro-benzol; 1-(β-Hydroxyethyl)amino-2-nitrobenzol; 4-(β-Hydroxyethyl)amino-3-nitrobenzamid; 1-(β-Hydroxyethyl)amino-4-N,N-bis(β-hydroxyethyl)amino-2-nitro-benzol; 1-(γ-Hydroxypropyl)amino-4-N,N-bis(β-hydroxyethyl)amino-2-nitrobenzol; 1-(β-Hydroxyethyl)amino-4-(N-methyl-N-β-hydroxyethyl)-amino-2-nitro-benzol; 1-(β-Hydroxyethyl)amino-4-(N-ethyl, N-β-hydroxyethyl)amino-2-nitro-benzol; 1-(β,γ-Dihydroxypropyl)amino 4-(N-ethyl-N-β-hydroxyethyl)amino-2-nitro-benzol; Disperse Orange 3; Disperse Red 17; Disperse Violet 4; Disperse Violet 8; Disperse Blue 1; Disperse Red 15; Solvent Violet 13; Solvent Violet 11; Disperse Blue 3; Disperse Blue 7; Disperse Red 11; Natural Brown 7; Disperse Black 9; Disperse Violet 15; Natural Orange 6; 2-Hydroxy 3-methoxy-1,4-naphthochinon; 3-Hydroxy-2-methyl-1,4-naphthochinon oder Phtiocol; 3,6-Dihydroxy-5-methoxy-p-toluchinon oder Spinulosin; HC Blue 14.

6. Zusammensetzung nach einem der vorhergehenden Ansprüche, wobei die Konzentration der anionischen und/oder nichtionischen Farbstoffe im Bereich von 0,0001 bis 10 Gew.-% des Gesamtgewichts der Zusammensetzung liegt.

7. Zusammensetzung nach einem der vorhergehenden Ansprüche, bei der die inerte(n) organische(n) Flüssigkeit(en) unter den Polydecenen der Formel C₁₀ₙH_{[(20n)+2]}, worin n im Bereich von 3 bis 9 liegt, den Estern von Fettalkoholen oder Fettsäuren, Estern oder Diestern von Zuckern und Fettsäuren mit 12 bis 24 Kohlenstoffatomen, cyclischen Ethern oder cyclischen Estern, Siliconölen, Mineralölen, pflanzlichen Ölen und deren Gemischen ausgewählt sind.

8. Zusammensetzung nach Anspruch 7, bei der die inerte(n) organische(n) Flüssigkeit(en) unter den Polydecenen der Formel C₁₀ₙH_{[(20n)+2]}, worin n im Bereich von 3 bis 9 liegt, den Estern von Fettalkoholen oder Fettsäuren und deren Gemischen ausgewählt sind.

9. Zusammensetzung nach einem der vorhergehenden Ansprüche, wobei die Konzentration der inerten organischen Flüssigkeiten im Bereich von 5 bis 60 Gew.-% des Gesamtgewichts der Zusammensetzung liegt.

10. Zusammensetzung nach einem der vorhergehenden Ansprüche, bei der das oder die Peroxysalz(e) unter den Persulfaten, Perboraten, Percarbonaten und Peroxiden von Alkalimetallen und Erdalkalimetallen und deren Gemischen ausgewählt sind.

11. Zusammensetzung nach Anspruch 10, wobei das oder die Peroxysalz(e) unter den Persulfaten und deren Gemischen ausgewählt sind.

12. Zusammensetzung nach Anspruch 11, wobei das oder die Peroxysalz(e) unter Natriumpersulfat, Kaliumpersulfat, Ammoniumpersulfat und deren Gemischen ausgewählt sind.

13. Zusammensetzung nach einem der vorhergehenden Ansprüche, bei der die Konzentration der Peroxysalze im Bereich von 1 bis 70 Gew.-% des Gesamtgewichts der Zusammensetzung liegt.

14. Zusammensetzung nach einem der vorhergehenden Ansprüche, bei der die Base(n) unter Harnstoff, Ammoniumchlorid, Ammoniumsulfat, Ammoniumphosphat, Ammoniumnitrat, Silicaten, Phosphaten oder Carbonaten von Alkali- oder Erdalkalimetallen und deren Gemischen ausgewählt sind.

15. Zusammensetzung nach einem der vorhergehenden Ansprüche, wobei die Konzentration der Basen im Bereich von 0,01 bis 40 Gew.-% des Gesamtgewichts der Zusammensetzung liegt.

16. Zusammensetzung nach einem der vorhergehenden Ansprüche, **dadurch gekennzeichnet, dass** sie wasserfrei ist.

17. Zusammensetzung nach einem der 1 bis 15 Ansprüche, **dadurch gekennzeichnet, dass** sie wässrig ist.

18. Zusammensetzung nach Anspruch 17, die ferner Wasserstoffperoxid enthält.

19. Verfahren zum Entfärben und gleichzeitigen Färben von Keratinfasern, **dadurch gekennzeichnet, dass** auf die Keratinfasern eine wasserfreie Zusammensetzung, wie sie in Anspruch 16 definiert ist, in Gegenwart einer wässrigen Zusammensetzung aufgebracht wird, die gegebenenfalls Wasserstoffperoxid enthält.

20. Vorrichtung mit mehreren Abteilungen, **dadurch gekennzeichnet, dass** sie mindestens zwei Zusammensetzungen enthält, deren Vermischen zu einer wässrigen Zusammensetzung führt, wie sie in Anspruch 17 oder 18 definiert ist.

21. Verwendung einer Zusammensetzung, wie sie in einem der Ansprüche 1 bis 18 definiert ist, zum Entfärben und gleichzeitigen Färben.
